# EUROPEAN PATENT APPLICATION

(11) **EP 1 323 706 A1**
(43) Date of publication of application: **02.07.2003**
(21) Application number: 02028993.0
(22) Date of filing: 27.12.2002
(51) Int. Cl.: C07C 213/04, C07D 251/34, C07C 231/12

(54) **Process for producing N-hydroxyalkyl compound, and tris(2-hydroxyethyl) isocyanurate composition**

(30) Priority: 28.12.2001 JP 2001399983
(71) Applicant: Nippon Shokubai Co., Ltd., Osaka-shi, Osaka 541-0043 (JP)
(72) Inventor: Takehiko, Morita, Ibaraki-shi, Osaka 567-0023 (JP); Toshimitsu, Moriguchi, Takatsuki-shi, Osaka 569-0854 (JP)
(74) Representative: Brehm, Hans-Peter

(57) **Abstract**

It is an object of the present invention to provide a process for producing an N-hydroxyalkyl compound which is not only conducive to suppression of the discoloration due to decomposition of the solvent and suppression of formation of impurities originating from the catalyst but also insures a high selectivity of reaction and a tris(2-hydroxyethyl) isocyanurate composition suppressed in discoloration, lean in impurity, with high resistance to heat and, hence, of good quality.

The present invention is directed to a process for producing an N-hydroxyalkyl compound comprising the reaction of an amido or imido group-containing compound and an epoxy compound in the presence of a catalyst, wherein the catalyst is a solid ion exchanger.

## Description

### FIELD OF THE INVENTION

The present invention relates to a processs for producing an N-hydroxyalkyl compound and a tris(2-hydroxyethyl) isocyanurate composition.

### DESCRIPTION OF THE RELATED ART

N-hydroxyalkyl compounds are industrially important compounds as starting materials for polyester resins, polyurethane resins, oxazolidone, textile treating agents, plasticizers, etc. or as solvents. Among such N-hydroxyalkyl compounds, tris(2-hydroxyethyl) isocyanurate [abbreviation: THEIC] which has hydroxyethyl groups on all the nitrogen atoms of a triazine ring, is not only excellent in heat resistance and flame retardancy due to the triazine ring but also has good compatibility so that it is of great use as a raw material for insulating varnishes, various coating resins, a stabilizer for vinyl chloride resins, or a flame retardant for various plastics.

Japanese Kokoku Publication Sho-43-26862 discloses a process for producing tris(2-hydroxylethyl) isocyanurate which comprises introducing ethylene oxide for reaction with cyanuric acid. Since the reactant cyanuric acid is hardly soluble in the common solvent, N,N-dimethylformamide (DMF) is used in the above process. However, DMF is decomposed during the reaction to contaminate the product compound, with the result that the product is discolored by impurity, thus detracting from the quality of the product. Incidentally, while the reaction according to this process is carried out in DMF without the aid of a catalyst, it is suspected that the solvent DMF has mild catalyst activity.

Japanese Kokoku Publication Sho-44-23332 discloses a process for producing a hydroxyalkyl isocyanurate which comprises the reaction of cyanuric acid and an alkylene oxide using a tertiary amine or a quaternary ammonium hydroxide as the catalyst in an aliphatic nitrile solvent containing not more than 5 carbon atoms. In consideration of the poor solubility of the starting material isocyanuric acid in the solvent, a catalyst is used in this process to make up for the retardation of the reaction but the impurity originating from the catalyst tends to contaminate the product and lower its quality. Moreover, some provision must be made for adding the alkylene oxide to all the nitrogen atoms of cyanuric acid to attain an improved selectivity.

JP Kokoku Publication Sho-45-8865 discloses a process for synthesizing a tris(β-hydroxyalkyl) isocyanurate which comprises the reaction of cyanuric acid in aqueous suspension and an alkylene oxide using an alkali metal or alkaline earth metal hydroxide or salt or a metal halide as the catalyst. However, in this synthetic process, too, the impurity originating from the catalyst tends to contaminate the product and interfere with the use of the product in various applications or otherwise detract from the quality of the product, and a further contrivance for improvement is required in this respect.

### SUMMARY OF THE INVENTION

The present invention, developed in the above state of the art, has for its object to provide a process for producing an N-hydroxyalkyl compound which is not only conducive to suppression of the discoloration due to decomposition of the solvent and suppression of formation of impurities originating from the catalyst but also insures a high selectivity of reaction and a tris(2-hydroxyethyl) isocyanurate composition suppressed in discoloration, lean in impurity, with high resistance to heat and, hence, of good quality.

The inventors of the present invention explored into the technology for producing an N-hydroxyalkyl compound which comprises the reaction of an amido or imido group-containing compound and an epoxy compound in the presence of a catalyst and found that in the case where the amido or imido group-containing compound is a solid which is hardly soluble in the common solvent, the use of a strongly polar solvent such as DMF or of a catalyst for making up for the retardation of the reaction owing to the poor solubility in the solvent allows the reaction to proceed efficiently between the active hydrogen of the amido or imido group and the epoxy compound. However, when a highly polar solvent is used, the solvent is decomposed to discolor the product and when a catalyst is used, the catalyst contaminates the product, with the result that troubles are encountered in the use of the N-hydroxyalkyl compound. However, the use of a solid ion exchanger leads to the advantage (1) that because it is a solid heterogeneous catalyst, there is no product contamination owing to the catalyst, (2) that because the reaction is accelerated by the catalyst (high activity), it is possible to liberally select a solvent without the necessity to select a solvent dissolving the starting material by force, for example a highly polar solvent such as DMF, (3) that the selectivity of addition of the epoxy compound to the amido or imido group is increased, and (4) that the reaction at low temperature becomes feasible. The present invention has been developed on the basis of the above findings.

The present invention, therefore, is directed to a process for producing an N-hydroxyalkyl compound comprising the reaction of an amido or imido group-containing compound and an epoxy compound in the presence of a catalyst, wherein the catalyst is a solid ion exchanger.

### DETAILED DESCRIPTION OF THE INVENTION

The present invention is now described in detail.

The process for producing an N-hydroxyalkyl compound according to the present invention comprises the reaction of an amido or imido group-containing compound and an epoxy compound, that is to say by reacting the active hydrogen of the amido or imido group with the epoxy compound, to give an N-hydroxyalkyl compound. These raw materials may each be used either singly or in a combination of two or more species. The preferred product N-hydroxyalkyl compounds of the present invention are tris(hydroxyalkyl) isocyanurates, and tris (2-hydroxyethyl) isocyanurate, which is useful for various applications, is particularly preferred. Moreover, because the impurity is particularly sparse as the effect of the present invention, a product of high heat resistance and high quality can be obtained.

The amido or imido group-containing compound mentioned above is preferably a cyclic amide or a cyclic imide. The cyclic amide is preferably isocyanuric acid, 2-pyrrolidone, ε-caprolactam or hydantoin, while the cyclic imide is preferably succinimide. Among these, isocyanuric acid is used for the production of tris(2-hydroxyethyl) isocyanurate. As the amino or imido group-containing compound, a straight-chain or a branched-chain compound can be used. Moreover, in the case of a compound showing tautomerism, it is sufficient that either one of the isomers has an amido group or an imido group. The ureido and ureylene groups derived from urea are also subsumed in the concept of amido or imido group in the context of the present invention.

The preferred epoxy compound includes aliphatic alkylene oxides such as ethylene oxide, propylene oxide, isobutylene oxide, 1,2-butylene oxide, 2,3-butylene oxide, pentylene oxide, etc.; aromatic alkylene oxides such as styrene oxide etc.; and cyclohexene oxide, among others. Among these, aliphatic alkylene oxides containing 2 to 3 carbon atoms, namely ethylene oxide and propylene oxide, are preferred. The most preferred is ethylene oxide.

As regards the ratio of the epoxy compound to the amido or imido group-containing compound, the number of mols of the epoxy group per mol of the amino or imido group is preferably not less than 0.5 mol and not more than 3 mols. If the proportion is less than 0.5 mol, the productivity of the N-hydroxyalkyl compound tends to be sacrificed. If it exceeds 3 mols, the selectivity tends to be decreased. The more preferred proportion is not less than 0.8 mol and the still more preferred proportion is not less than 0.9 mol. It is more preferably not more than 1.5 mols, still more preferably not more than 1.2 mols.

The reaction time can be judiciously selected according to the degree of crushing of the amido or imido group-containing compound, e.g. isocyanuric acid, type of catalyst, reaction temperature, reaction molar ratio, and kind of solvent, among other variables. The preferred reaction time is 0.1 to 12 hours.

In the present invention, a solvent may be optionally used or not used but is preferably used in cases where the melting point of the amido or imido group-containing compound is higher than the reaction temperature or the same compound has no definite melting point of its own. In such cases, a suitable solvent can be judiciously selected with reference to the properties of the amido or imido group-containing compound.

In the present invention, a solid ion exchanger is used. The solid ion exchanger is what is generally known as a heterogeneous catalyst and such that its ion exchange groups functioning as active sites are not released homogeneously into the reaction system but are fixed to show an ion exchange capacity. As such ion exchangers, it is possible to use hydrotalcite, zeolite, hydroxyapatite, silica gel, alumina gel, aluminosilica gel, clay minerals, and ion exchange resin catalysts. Among these, ion exchange resin catalysts are preferred in view of their high catalyst activity.

The ion exchange resin catalyst referred to above may be any macromolecular compound having an ion exchange capacity but is particularly preferably a strongly basic anion exchange resin or a weakly basic anion exchange resin, etc.

In this specification, the term "macromolecular compound" is used to globally mean any and all polymers and compounds having molecular weight distributions and does not imply any particular molecular weight range.

The ion exchange resin catalyst for use in the present invention preferably contains quaternary ammonium salt structures, more preferably cyclic quaternary ammonium salt structures. A suitable ion exchange resin catalyst of this type preferably comprises an organic macromolecular compound essentially having a main chain, preferably main chains and crosslinking sites, said main chain or chains predominantly comprising of a repeating unit having a quaternary ammonium structure. More preferably it comprises an organic macromolecular compound comprising of a repeating unit having a cyclic amine structure, namely an organic macromolecular compound predominantly comprising a repeating unit having a cyclic quaternary ammonium salt structure. Preferably this quaternary ammonium salt structure is such that a cationized nitrogen atom has been incorporated into the main chain via a couple of bonds or into the crosslinking site via 4 bonds.

The compound may additionally contain a repeating unit not having a quaternary ammonium salt structure. The organic macromolecular compound means a polymer compound having a main chain structure predominantly composed of hydrocarbons. The main chain constitutes a skeleton in the organic macromolecular compound. When the organic macromolecular compound has a main chain(s) and a crosslinking site(s) as essential constituents, the compound is referred to sometimes as a crosslinked organic polymer.

Since the cyclic amine structure mentioned above is less susceptible to oxidative decomposition than an aliphatic amine structure, this ion exchange resin catalyst is more resistant to thermal degradation. Such the cyclic amine structure is preferably a 5- or 6-membered, more preferably a 5-membered ring. The diallyldimethylammonium salt structure is particularly preferred because it facilitates formation of a main chain predominantly comprising a repeating unit having said cyclic quaternary ammonium salt structure.

The ion exchange resin catalyst mentioned above preferably comprises an organic macromolecular compound essentially having a hetero atom in a main chain(s) and/or a crosslinking site(s) and any of the hetero atom in a main chain(s) and/or a crosslinking site(s) does not have a hydrogen atom attached to it.

The organic macromolecular compound mentioned above has a structure having hetero atoms incorpolated into one or both of its main chain(s) and crosslinking site(s). That the compound essentially has hetero atoms incorporated into its main chain or chains and/or crosslinking site(s) means that it essentially contains hetero atoms incorporated into the main chain(s) and/or crosslinking site(s) and does not mean that any group containing a hetero atom is attached to the main chain(s) and/or crosslinking site(s) in the manner of a side chain. By insuring that said organic macromolecular compound should have such a structure, the heat resistance is improved and chemical degradation inhibited as compared with the case in which the active site occurs in the side chain, with the result that the active site of the ion exchange resin catalyst may not easily be released into the reaction system during production of the N-hydroxyalkyl compound.

Referring, further, to the organic macromolecular compound mentioned above, the crosslinking site means the site in which main chains are interconnected and this crosslinking site consists of moieties constituting part of the main chains and a crosslink structure interconnecting them. The main chain and the crosslink structure are usually coupled together by covalent bonding. The presence of such crosslinking sites contributes to an increased mechanical strength of the ion exchange resin catalyst.

The hetero atoms occurring in the main chain(s) and/or crosslinking site(s) of said organic macromolecular compound can function as active sites exhibiting catalyst activity in the reaction giving rise to the N-hydroxyalkyl compound from said amido or imido group-containing compound and epoxy compound. Thus, because the active sites of the organic macromolecular compound constituting the ion exchange resin catalyst have been built into the main chain(s) and/or crosslinking site(s) structure, the catalyst is structurally less susceptible to pyrolysis so that, compared with the conventional polymer catalyst, the active sites are less liable to be cleaved or decomposed by heat, for instance, during the reaction, thus making the catalyst a highly heat-resistant ion exchange resin catalyst.

The preferred hetero atom mentioned above includes nitrogen, oxygen, sulfur, phosphorus, aluminum, boron, zinc, copper, nickel, iron atoms, etc. These may occur one species consistently throughout or in a combination of two or more species. Among these, nitrogen, sulfur and phosphorus are preferred hetero atoms and nitrogen is most preferred. These hetero atoms preferably exist as ionized for improved catalyst activity. More preferably, they have been cationized. Therefore, the most desirable form of the ion exchange resin catalyst according to the invention has such a structure that the organic macromolecular compound constituting the ion exchange resin catalyst has cationized nitrogen atoms in its main chain(s) and/or crosslinking site(s), that is to say cationized nitrogen atoms have been built into the main chain(s) and/or crosslinking site(s). Referring to such a structure, in order that the proportion of active sites in the ion exchange resin catalyst may be increased for enhanced catalyst activity, it is preferable to insure that the cationized nitrogen atom has been built into the main chain by two bonds and/or that the cationized nitrogen atom has been built into the crosslinking site(s) by 3 or 4 bonds.

The organic macromolecular compound, as mentioned above, preferably has no hydrogen atom attached to the hetero atom in a main chain(s) and/or a crosslinking site(s).
If the organic macromolecular compound has a hydrogen atom attached to the hetero atom, this hydrogen atom will react with the epoxy compound to cause the ion exchange resin catalyst to swell with the progress of the reaction between said epoxy compound and amido or imido group-containing compound and thereby reduce the reaction efficiency and interfere with the repeated use of the ion exchange resin catalyst. The hydrogen atom attached to the hetero atom means the hydrogen atom directly attached to the hetero atom by covalent bonding, as in the case of -OH, -NH, -NH₂, -SH, or -COOH, etc, and coordinate bonding is not included. The hydrogen atom attached to the hetero atom contained in the polymerization initiator, suspending agent or dispersion stabilizer, etc is not included, either.

The above organic macromolecular compound preferably has a counter anion. The counter anion mentioned just above means an anion coordinated to the cationized hetero atom at a cationized site in the main chain(s) and/or crosslinking site(s). The number of such counter anions per molecule of the organic macromolecular compound is not particularly restricted.

The preferred counter anion is a halide ion, hydroxide ion, organic acid anion or inorganic acid anion, etc. The preferred halogen atom for said halide ion is fluorine, chlorine, bromine or iodine, etc. The organic acid anion or inorganic acid anion means the anion originating from an organic acid or inorganic acid upon removal of at least one hydrogen ion. The preferred inorganic acid anion includes sulfate ion, phosphonate ion (phosphite ion), borate ion, cyanide ion, carbonate ion, hydrogencarbonate ion, thiocyanate ion, thiosulfate ion, sulfite ion, hydrogensulfite ion, nitrate ion, cyanate ion, phosphate ion, hydrogenphosphate ion, metalate ion (e.g molybdate ion, tungstate ion, metavanadate ion, pyrovanadate ion, hydrogenpyrovanadate ion, niobate ion, tantalate ion, perrhenate ion, etc.), tetrafluoroaluminate ion, tetrafluoroborate ion, hexafluorophosphate ion, tetrachloroaluminate ion, Al₂Cl₇⁻, and so on. The preferred organic acid anion includes sulfonate ion, formate ion, oxalate ion, acetate ion, (meth)acrylate ion, trifluoroacetate ion, trifluoromethanesulfonate ion, bis(trifluoromethanesulfon)amide ion, (CF₃SO₂)₃C⁻, and so on. These counter anions may be present one species only or in a combination of two or more species in the organic macromolecular compound. The preferred, among these, are halide, hydroxide, hydrogencarbonate, hydrogensulfite, formate, and metalate ions. The more preferred are chloride ion, a species of said halide ion, hydroxide ion, hydrogencarbonate ion, and formate ion. The most preferred is hydroxide ion in the use of the catalyst or chloride ion in the synthesis of the catalyst. Therefore, in the present invention, hydroxide ion is preferably an essential counter anion. This is conducive to enhanced catalyst activity. Thus, while in the case where the compound contains two amido or imido groups, one mol of the epoxy compound is added to each of the groups to give a 2-mol adduct or in the case where the compound contains three amido or imido groups, one mol of the epoxy compound is added to each of the groups to give a 3-mol adduct, the selectivity for each particular adduct structure is further improved under the above conditions.

In the case where said organic macromolecular compound has a crosslinking sites, the structure of the crosslinking site is not particularly restricted and the number of such sites is not particularly restricted, thus being one or more in the organic macromolecular compound. In cases where two or more crosslinking sites are present per mol of the organic macromolecular compound, all the crosslinking sites may be of the same structure or of different structures. The structure of such an organic macromolecular compound having crosslinking site(s) is usually a network structure consisting of main chains and crosslinking site(s).
The average number of main chains per mol of the organic macromolecular compound is not less than 2 but is otherwise not particularly restricted.

Regarding the crosslink density of said organic macromolecular compound having a crosslinking site(s), the proportion of the monomer (crosslinking agent) constituting the crosslink structure relative to the total number of mols of the or monomer(s) constituting the organic macromolecular compound is preferably not less than 0.1 mol % and not more than 80 mol %. If the proportion is less than 0.1 mol %, the mechanical strength of the ion exchange resin catalyst tends to be too low. If it exceeds 80 mol %, the permiability of the reactant solution through the ion exchange resin catalyst during production of the N-hydroxyalkyl compound tends to be sacrificed so that the catalyst activity may not be sufficiently expressed. The proportion is more preferably not less than 0.5 mol %, still more preferably not less than 1 mol % and is more preferably not more than 50 mol %, still more preferably not more than 20 mol %.

The structure of said crosslinking site is preferably the structure represented by the following general formula (1) and/or the structure represented by the following general formula (2). 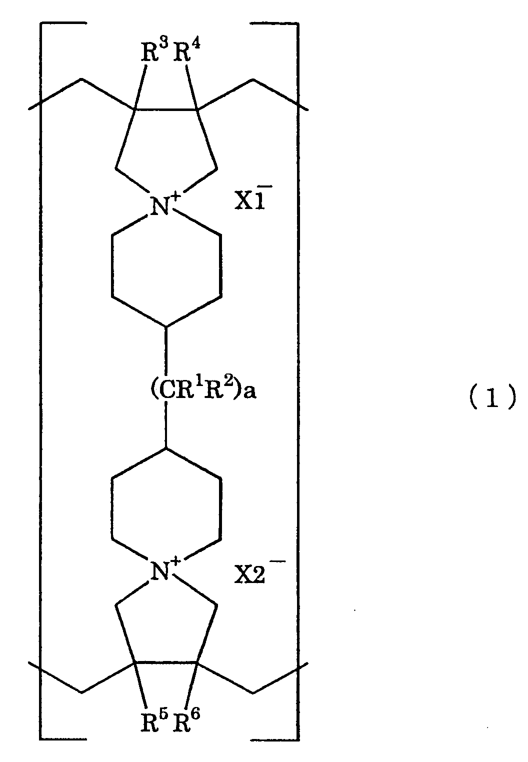 Referring to the general formula (1), R¹ and R² may be the same or different and each represents a hydrogen atom, a halogen atom, an alkyl group containing 1 to 10 carbon atoms, or a hydroxyl group; R³, R⁴, R⁵ and R⁶ are the same or different and each represents a hydrogen atom, a halogen atom, a methyl group, or an ethyl group; X1⁻ and X2⁻ represent counter anions, which may be the same or different and each represents a halide ion, a hydroxide ion, an organic acid anion or an inorganic acid anion; a represents an integer of 0 to 10. 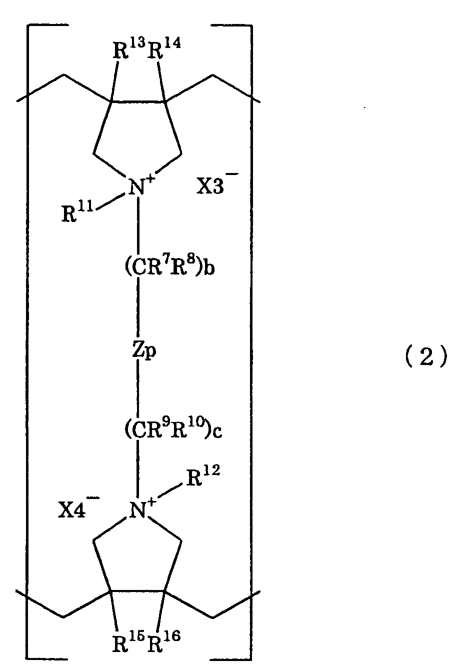 Referring to the general formula (2), R⁷, R⁸, R⁹ and R¹⁰ are the same or different and each represents a hydrogen atom, a halogen atom, an alkyl group containing 1 to 10 carbon atoms, or a hydroxyl group; R¹¹ and R¹² are the same or different and each represents an alkyl group containing 1 to 10 carbon atoms; R¹³, R¹⁴, R¹⁵ and R¹⁶ are the same or different and each represents a hydrogen atom, a halogen atom, a methyl group, or an ethyl group; X3⁻ and X4⁻ are counter anions, which are the same or different and each represents a halide ion, a hydroxide ion, an organic acid anion, or an inorganic acid anion; b and c are the same or different and each represents an integer of 0 to 10; p represents 0 or 1, with the proviso that the relation b + c + p ≥ 1 is satisfied; Z represents -NH-, -N(CH₃)-, -NH-(CH₂)₃-NH-, -NH-(CH₂)₄-NH-, -O-, -CH(OH)-, -O-CH₂-C(CH₃)₂-CH₂-O-, -O-(CH₂)₂-(O-CH₂-CH₂)ₙ-O-, 1,4-piperazinylene, 3-methyl-2,6-pyridyl, 4-methyl-2,6-pyridyl, 2,6-pyridyl, or 2,5-pyridyl; n represents an integer equal to not less than 0.

The above crosslinking site structure is more preferably such that, in the above general formula (1), R¹ to R⁶ invariably represent hydrogen atom; X1⁻ and X2⁻ represent chloride ion and/or hydroxide ion; and a is equal to 3, that is the structure assumed by N,N,N',N'-tetraallyldipiperidylpropanium dichloride, or the corresponding structure available upon substitution of hydroxide ions for chloride ions.

The following is a production method of a preferred ion exchange resin catalyst for use in the process for producing an N-hydroxyalkyl compound according to the present invention. This preferred ion exchange resin catalyst comprises an organic crosslinked polymer obtainable by subjecting an aqueous solution containing an ammonium compound of the following general formula (3) and a compound having at least 2 diallylammonium groups within the molecule to copolymerization reaction in a water-insoluble organic solvent by the reversed phase suspension polymerization or reversed phase emulsion polymerization technique. Referring to such an organic crosslinked polymer, the compound having 2 or more diallylammonium groups within the molecule serves as a crosslinking agent to form a crosslink structure and the crosslinked polymer is obtained as spherical particles, i.e. beads. The ion exchange resin catalyst of the present invention may also be obtained by subjecting said aqueous solution to solution polymerization and molding or granulating the resulting polymer into the desired shape. 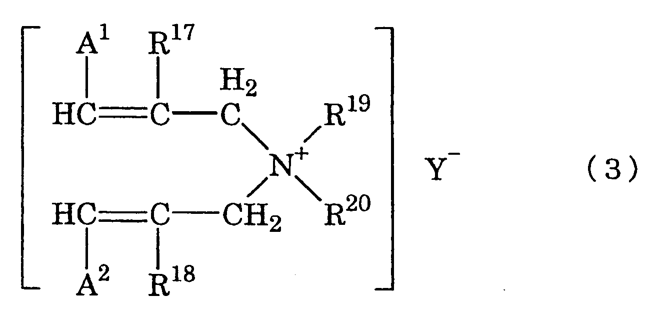 Referring to the general formula (3), R¹⁷ and R¹⁸ are the same or different and each represents a hydrogen atom, a methyl group, an ethyl group, or a halogen atom; R¹⁹ and R²⁰ are the same or different and each represents an alkyl group, an alkoxyalkyl group, or an aryl group or R¹⁹-N⁺-R²⁰ represents a piperidine ring or a morpholinium ring as formed by the coupling of R¹⁹ and R²⁰; A¹ and A² are the same or different and each represents a hydrogen atom, an alkyl group, or a halogen atom; Y⁻ represents a counter anion.

The halogen atom mentioned above is preferably fluorine, chlorine, bromine, iodine or the like. The alkyl or alkoxyalkyl group mentioned above is preferably a group whose total number of carbon atoms is 1 to 18. Among such preferred alkyl groups are methyl, ethyl, n-propyl, isopropyl, n-butyl, isobutyl, tert-butyl, pentyl, hexyl, heptyl, octyl, nonyl, decyl, undecyl, dodecyl, tridecyl, tetradecyl, pentadecyl, hexadecyl, heptadecyl, and octadecyl, etc. The more preferred are the groups which contain 1 to 12 carbon atoms; the still more preferred are those groups which contain 1 to 8 carbon atoms; and the most preferred groups are those containing 1 to 6 carbon atoms.

The aryl group mentioned above is preferably a group containing 6 to 15 carbon atoms, more preferably a group of 6 to 12 carbon atoms. Among such preferred aryl groups are phenyl, tolyl and xylyl, etc.

Referring to the piperidine ring or morpholinium ring mentioned above, one or more of its hydrogen atoms may be substituted by alkyl group containing 1 to 13 carbon atoms.

The ammonium compound represented by the above general formula (3) includes diallyldialkylammonium salts such as diallyldimethylammonium salts, diallyldiethylammonium salts, etc.; and dimethalyldialkylammonium salts such as dimethalyldimethylammonium salts, etc. to mention a few preferred compounds. Diallyldimethylammonium salts are preferred because, with these, it is easy to form a main chain predominantly consisting of a repeating unit having a quaternary ammonium salt structure. These may be used each independently or in a combination of two or more species.

As preferred examples of said compound having two or more diallylammonium groups within the molecule, there can be mentioned the compounds represented by the following formulas (1) to (10) and the like.

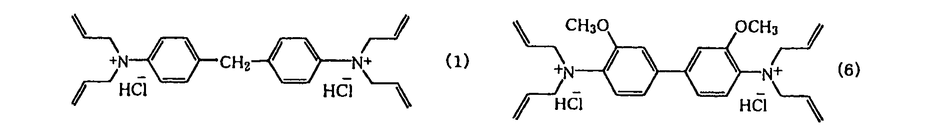

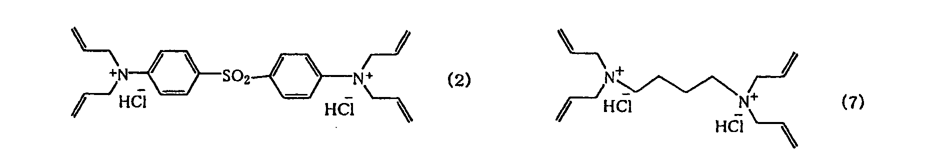

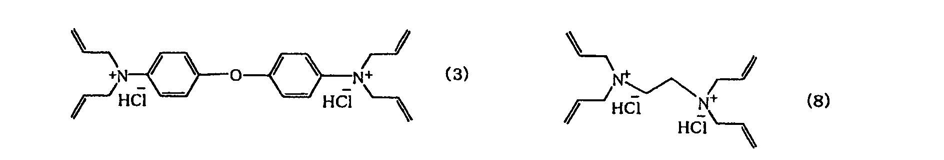

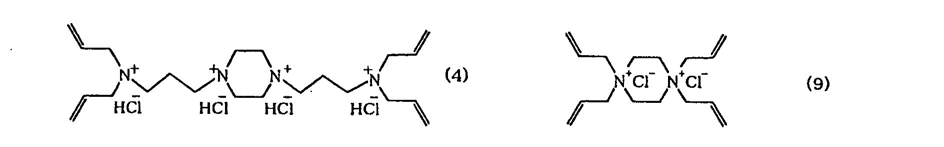

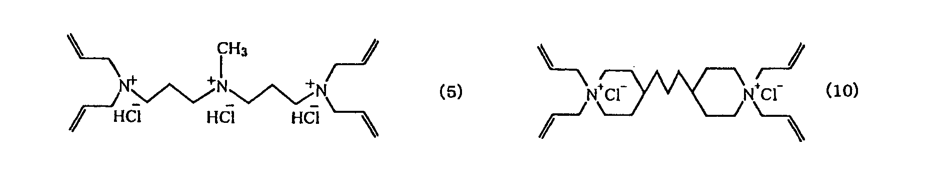

Among the compounds represented by the above formulas (1) to (10), N,N,N',N'-tetraallyldipiperidylpropanium dichloride of the formula (10) is still more preferred. As said compound having 2 or more diallylammonium groups within the molecule other than the above compounds of formulas (1) to (10), N,N'-dimethyl-N,N,N',N'-tetraallyl-2-butene-1,4-diammonium dichloride can be mentioned. Also, as compounds having 2 or more p-vinylphenylmethylammonium and/or m-vinylphenylmethylammonium groups, N,N'-di(p-vinylphenylmethyl)ethylenediammonium dichloride, N,N'-di(m-vinylphenylmethyl)ethylenediammonium dichloride, N,N'-di(p-vinylphenylmethyl)propylenediammonium dichloride, N,N'-di(m-vinylphenylmethyl)propylenediammonium dichloride and the like can be used with advantage. The above are representative examples, and aside from these, compounds which can be synthesized from a diamine such as phenylenediamine and allyl chloride or allyl acetate are also preferred. These can be used each independently or in a combination of two or more species. It should be understood that the counter anions possessed by these compounds need not be chloride anions but may be other anions.

For the production of said organic crosslinked polymer, the copolymerizing molar ratio of said ammonium compound to said compound having two or more diallylammonium groups within the molecule is preferably 40/60 to 99.9/0.1. Compounds other than said compounds may also be copolymerized in suitable amounts. Moreover, regarding the concentration of the compound in aqueous solution, the concentration of the solution in charging is preferably 30 to 80 mass % for handling reason but such an aqueous solution may be concentrated by a dehydration procedure before the start of polymerization or during the polymerization reaction.

The preferred water-insoluble organic solvent for use as said medium includes toluene, xylene, benzene, n-hexane, cyclohexane, octane, mineral acids, mineral spirit, kerosine, and brominated and/or chlorinated hydrocarbons such as 1,1,1-trichloroethane, 1,2-dichloropropane, tetrachloroethane, trichloropropane, tetrachloromethane, and so on. These may be used each independently or in a combination of two or more species. Among these, toluene or xylene is industrially advantageous.

The medium mentioned above may contain a viscous fluid such as liquid paraffin and/or silicone oil, etc in which case it becomes possible to inhibit aggregation and adhesion of the beads to prevent blocking of the organic crosslinked polymer. The proportion of such a viscous fluid in the medium is preferably 0.1 to 50 mass %, more preferably 1 to 40 mass %, still more preferably 5 to 30 mass %. As regards the viscosity of the medium containing such a viscous fluid, it is only sufficient that addition of the fluid has resulted in an increase in viscosity.

The polymerization conditions of said copolymerization, namely polymerization temperature, polymerization time, stirring conditions, the polymerization initiator and additives to be used, and among other variables, can be judiciously selected with reference to the expected size, properties, and quality of beads, among other factors. The polymerization temperature, for instance, is preferably 20 to 150°C, more preferably 50 to 120°C. The reaction can be carried out either at atmospheric pressure or under reduced pressure, optionally along with azeotropic dehydration. The polymerization time can be selected with reference to the other conditions used and is preferably somewhere between 4 and 50 hours. As to the stirring conditions, the stirring rate for usual polymerization can be used. As the polymerization initiator to be used, a conventional peroxide type or azo type initiator can be used, irrespective of whether it is water-soluble or oil-soluble. As said additives, the conventional dispersion stabilizer, such as glycerol palmitate, ethylcellulose, sorbitan monopalmitate, sorbitan monolaurate, sorbitan monostearate, or the like, for instance, can be used.

As a representative example of the process for producing said organic crosslinked polymer, the process comprising adding an aqueous solution of a diallyldimethylammonium salt and N/N/N'/N'-tetraallyldipiperidylpropanium dichloride and an aqueous solution of said polymerization initiator dropwise to a medium composed of toluene and liquid paraffin supplemented with a dispersion stabilizer and carrying out a reversed phase suspension polymerization or reversed phase emulsion polymerization reaction is depicted in the following reaction scheme (1).

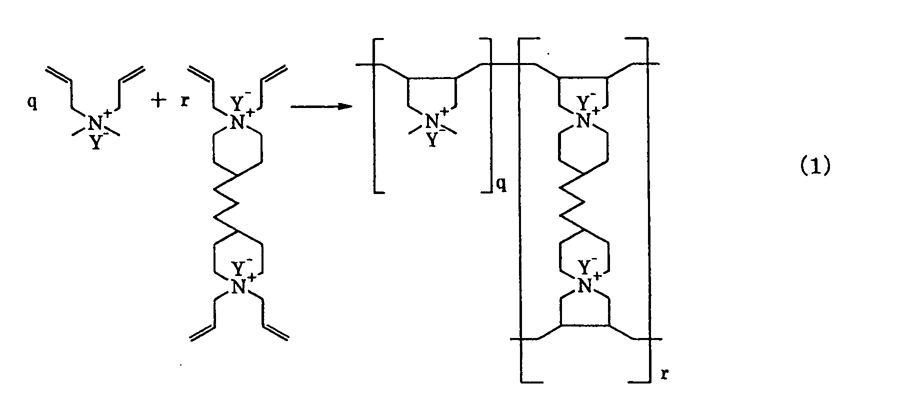

In the above reaction scheme (1), Y⁻ represents chloride ion as the counter anion; q and r each represents the number of mols.

The mean particle diameter of the beads obtainable by the above reversed phase suspension polymerization or reversed phase emulsion polymerization is preferably set at 0.05 to 5 mm.

Referring to the process for producing an N-hydroxyalkyl compound according to the present invention, the level of use of the ion exchange resin catalyst, for instance, the mode of feeding it to the reactor, and the process for separating the ion exchange resin catalyst from the reaction mixture after completion of the reaction can be judiciously selected according to the mode of practicing the production method of the present invention, etc. Regarding the reaction conditions, the reaction temperature is preferably 50 to 180°C. If it is below 50°C, the reaction rate slows down to detract from the yield per unit time. If the reaction temperature exceeds 180°C, the selectivity for the objective N-hydroxyalkyl compound, particularly the 3-mol adduct, tends to be decreased. The more preferred reaction temperature is 70 to 140°C and the still more preferred temperature is 80 to 120°C. The reaction pressure is preferably 0.1 to 3 MPa, more preferably 0.15 to 2 MPa, still more preferably 0.2 to 1 MPa.

The process for producing an N-hydroxyalkyl compound in accordance with the present invention may be carried into practice in the presence of carbon dioxide or an inert gas such as nitrogen, argon, helium or the like. Moreover, when hydroxide ions are used as the counter anions, the catalyst may be regenerated after the reaction by treating it with a hydroxyl group-containing basic solution appropriately for the maintenance of catalyst activity necessary for the reaction and compensation for loss of the anions. Moreover, insofar as the operation and result of the present invention may be achieved, it is permissible, where necessary, to add a different catalyst, for instance, to the reaction system in addition to the starting epoxy compound and amido or imido group-containing compound mentioned above and the above-mentioned catalyst.

In the process for producing an N-hydroxyalkyl compound according to the present invention, the epoxy compound is reacted with the amido or imido group-containing compound in the presence of the catalyst (ion exchange resin catalyst) but the catalyst activity of this catalyst is so high that the discoloration due to decomposition of the solvent is suppressed and, moreover, since the heat resistance of the catalyst is high, the impurity originating from the catalyst is also suppressed. Furthermore, the selectivity for the N-hydroxyalkyl compound is high. The catalyst for use is preferably treated with a liquid containing the solvent to be used at 50 to 180°C for 0.1 to 24 hours. This treatment contributes to greater stabilization of product quality. In the case where recrystallization is required, the process can be simplified by using the same solvent for the reaction solvent and the recrystallization solvent. Moreover, since the ion exchange resin catalyst is formed into spherical particles as described above, it is easy to separate the ion exchange resin catalyst from the reaction product, making the process more suited for commercial exploitation. Moreover, the reaction using said ion exchange resin catalyst can be carried out in a stirring batch reactor or a fixed bed or fluidized bed reactor, for instance, that is to say whether batchwise or continuously. In addition, the catalyst can be used for reactive distillation wherein the catalyzed reaction and the purification of the reaction product can be efficiently carried out. Thus, the mode of use is by no means restricted by the kind of process.

By the above-described production method, using isocyanuric acid as the amido or imido group-containing compound and ethylene oxide as the epoxy compound, a tris(2-hydroxyethyl) isocyanurate composition lean in catalyst- and solvent-derived impurities and high in heat resistance can be produced in accordance with the reaction illustrated in the reaction scheme (2) presented below. The tris(2-hydroxyethyl) isocyanurate composition obtainable by the above process is also an aspect of the present invention. The high heat resistance referred to above means that when the product is subjected to thermal analysis, the loss of mass at an ultimate temperature of 300°C is not greater than 55%, preferably not greater than 40%, more preferably not greater than 20%, most desirably not greater than 10%. The tris(hydroxyethyl) isocyanurate (THEIC) composition obtainable by the process is very advantageous, for it does not give rise to any appreciable amount of 2-oxazolidone upon heating, is low in viscosity after heating and, furthermore, is easy to handle when used in applications calling for heat resistance.

The composition according to the present invention may contain a secondary component in addition to THEIC. The THEIC content of the composition is not less than 10% at a minimum, preferably not less than 25%, more preferably not less than 50%, still more preferably not less than 90%, and most preferably not less than 95%.

The secondary component includes, among others, the starting materials and solvent used in the reaction, the reaction byproduct, and even a stabilizer added after the reaction.

The reaction byproduct includes isocyanuric acid-ethylene oxide adducts such as 1 mol ethylene oxide adduct, 2 mol ethylene oxide adduct, 4 mol ethylene oxide adduct, 5 mol ethylene oxide adduct, etc., 2-oxazolidone arising from decomposition of THEIC, the polymer formed as said 2-oxazolidone undergoes further reaction, the polyethylene oxide arising from ring-opening reaction of ethylene oxide, and various other components arising from the reaction of interest. Compounds other than those mentioned above that may form in the course of reaction are all subsumed in the concept of said secondary component.

The composition according to the present invention may, for example, be a solution available on removal of the solid catalyst after completion of the reaction, a precipitate (crude product) available on removal of the solvent following the reaction, or a purified preparation (product) as purified by a purity-enhancing procedure, for example by recrystallization, extraction, and drying of the crude product. It may be said crude product or product supplemented with a diluent, stabilizer and the like.

The slurry-like product containing precipitates due to poor solubility in the solvent is also subsumed in the concept of said composition.

As the product, one with a THEIC purity of not less than 95% is preferred. The more preferred purity is not less than 98%.

The high activity of the catalyst for use in the present invention permits a liberal selection of reaction solvent but when the same solvent is specified for both the reaction solvent and the recrystallization solvent, the recrystallization of the crude product after removal of the solvent from the reaction mixture may be omitted and the final product can be obtained by a mere drying procedure. From this point of view, the reaction solvent is preferably selected from among alcohols having 1 to 6 carbon atoms, such as methanol, ethanol, n-propanol, 2-propanol, n-butanol, hexanol, cyclohexanol, etc., glycol ethers containing 3 to 6 carbon atoms, such as methyl-cellosolve, ethyl-cellosolve, butyl-cellosolve, etc., and ethers containing 2 to 6 carbon atoms, such as tetrahydrofuran and dioxane.

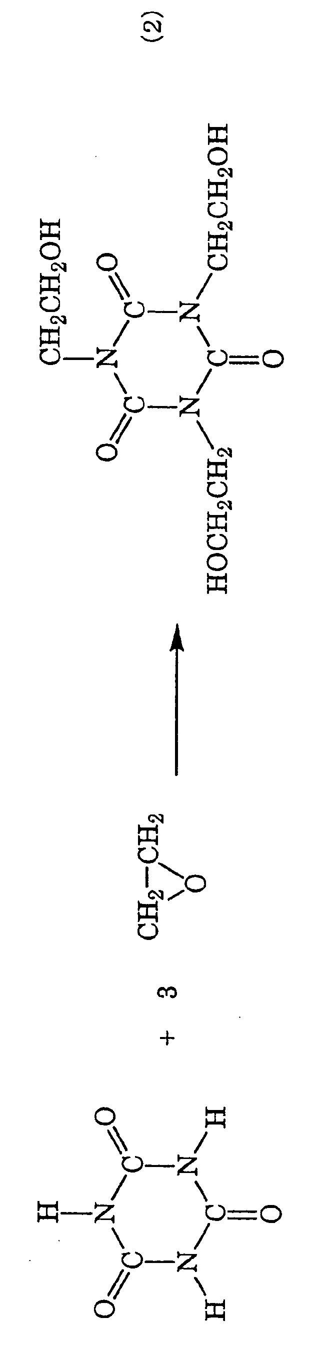

The tris(2-hydroxyethyl) isocyanurate composition mentioned above has high heat resistance and flame retardancy and, therefore, is a substance of great use in various fields such as electrically insulating vanishes and other electric conductor varnishes; stabilizers for vinyl chloride resin; raw materials for various coating resins, and flame retardants for various plastics, among others.

The process for producing an N-hydroxyalkyl compound according to the present invention which is constituted as above is capable of inhibiting the discoloration due to decomposition of the solvent and suppressing contamination with the impurity derived from the catalyst and, in addition, featuring a high selectivity for the objective N-hydroxyalkyl compound. By this method, therefore, N-hydroxyalkyl compounds finding a broad range of application such as raw materials for polyester resins, polyurethane resins, oxazolidone, textile-treating agents, plasticizers, etc. and solvents, etc. can be produced. Particularly, the method enables advantageous production of tris(2-hydroxyethyl) isocyanurate which can be used in a variety of applications such as a starting material for insulating varnishes and other electric conductor varnishes, stabilizers for vinyl chloride resin, raw materials for various coating resins, flame retardants for various plastics, and so on.

Furthermore, the invention provides a tris(2-hydroxyethyl) isocyanurate composition which has been suppressed in discoloration and impurity and improved in resistance to heat, hence being of high quality and great use in a broad range of application.

### BEST MODE FOR CARRYING OUT THE INVENTION

The following examples are further illustrative but by no means definitive of the present invention. The "%" in this specification means "mass %" unless otherwise specified. Catalyst preparation method 1

A 1L separable flask equipped with an anchor-shaped stirring blade, reflux condenser, thermometer, nitrogen gas inlet tube, and dropping funnel was charged with 350 mL of toluene and 50 mL (12.5 vol. % in medium) of liquid paraffin (product of Wako Pure Chemical Ind., special grade). Then, as dispersion stabilizers, 0.071 g of sorbitan monopalmitate and 0.213 g of ethylcellulose were added and dissolved. In this procedure, the dissolved oxygen was removed by blowing nitrogen gas into the flask.

Separately, 41.80 g of a 65% aqueous solution of diallyldimethylammonium chloride (DADMAC), 8.26 g of N,N,N',N'-tetraallyldipiperidylpropanium dichloride (TADPPC), and 5.36 g of water were admixed to prepare a solution, to which was added a solution prepared by mixing 0.319 g of the polymerization initiator "V-50" with 3.52 g of water (monomer concentration 59.8%, DADMAC/TADPPC = 90/10 mol %).

This mixture solution was added dropwise to the reaction vessel over 30 minutes under constant stirring at 220 rpm and, after completion of dropping, the reaction was carried out at 55°C for 4 hours, at 60°C for 16 hours, and further at 92 to 95°C for 6 hours. Meanwhile, at the point of time when 2 hours had elapsed, the stirring speed was increased from 220 rpm to 250 rpm and 50 mL (22.2 vol. % in medium) of liquid paraffin was added dropwise. Moreover, when the temperature was raised from 55°C to 60°C, 50 mL of toluene was further added (liquid paraffin concentration 20.0 vol % in medium).

After a predetermined time of reaction, the reaction mixture was cooled and the product particles were separated by filteration. The particles separated were washed with 600 mL of toluene and 800 mL of methanol for a total of 3 times and dried under reduced pressure at 60°C overnight to give 36.23 g of dried particles. The dried particles thus obtained were separated particles with a diameter of about 0.3 mm. A 30.0 g portion of the resulting crosslinked polymer carrying the chloride ion as the counter anion was caused to swell with water and the counter anion was substituted hydroxide ion for chloride ion by the column technique using 5 L of 2N aqueous sodium hydroxide solution. Thereafter, the product was washed with the same amount of water to give an ion exchanger having the structure shown in the above reaction scheme (1).

### Example 1

Using the ion exchanger obtained in Catalyst preparation method 1, the addition reaction of ethylene oxide to isocyanuric acid was carried out.

Specifically, an autoclave equipped with a thermometer, pressure gauge, stirrer, etc. was charged with 20 mL of the water-swollen ion exchanger, 9.7 g of isocyanuric acid, and 37.5 g of methyl-cellosolve, and 9.9 g of ethylene oxide was continuously introduced over 1 hour in a nitrogen atmosphere at 90°C. As the water-swollen ion exchanger, wet product, which was prepared by measuring with a graduated cylinder carrying out solvent substitution with methyl-cellosolve and suction-filteration, was used. The reaction mixture was then matured at 90 °C for 6 hours to synthesize tris(2-hydroxyethyl) isocyanurate. The methyl-cellosolve mentioned above was fed in such a manner that the total amount of methyl-cellosolve inclusive of the amount contained in the ion exchanger would be 37.5 g. From the colorless clear solution available on completion of the reaction, the ion exchanger was recovered by filtration and the filtrate was analyzed by high performance liquid chromatography. As so analyzed, the conversion rate of isocyanuric acid was 94.3 mol % and the yield of tris(2-hydroxyethyl isocyanurate was 71.9 mol %. From this solution, the methyl-cellosolve was recovered and the solution was cooled, whereupon white solid crystals separated out. The crystals were recovered by filtration and dried. The white solid crystal thus obtained was found to comprise tris(2-hydroxyethyl) isocyanurate of 99.0% purity.

### Example 2

Using the ion exchanger recovered by filtration in Example 1, a repeat experiment was performed in the same manner as in Example 1. From the colorless clear solution available on completion of the reaction, the ion exchanger was recovered by filtration and the filtrate was analyzed by high performance liquid chromatography. As so analyzed, the conversion rate of isocyanuric acid was 99.0 mol % and the yield of tris(2-hydroxyethyl) isocyanurate was 84.7 mol %. There was no activity drop of the catalyst due to the repeated catalyst reaction. From this solution, the methyl-cellosolve was recovered and the solution was cooled, whereupon white solid crystals separated out. This crystal was separated by filtration and dried. The resulting white solid crystal crop was found to comprise tris(2-hydroxyethyl) isocyanurate of 99.0% purity.

The crystals thus obtained were analyzed with a thermal analyzer (product of Shimadzu Corporation, DTG-50). In a nitrogen gas stream at 5 ml/min, the temperature was increased first to 200°C at 30°C/min and maintained at that level for 10 minutes. Then, the temperature was further increased to 250°C at 10°C/min and held at this level for 10 minutes. The temperature was further increased to 350°C at 10°C/min and held for 5 minutes. The loss of mass after 10 minutes' incubation at 250°C was 0.9% and the loss of mass after arrival at 300°C was 1.7%, indicating a good heat resistance.

### Example 3

Using the crosslinked polymer recovered by filtration in Example 2, 5 additional repeat experiments were performed in the same manner as in Example 2. From the colorless clear solution available upon completion of the 5th reaction, the crosslinked polymer was recovered by filtration and the filtrate was analyzed by high performance liquid chromatography. As so analyzed, the conversion rate of isocyanuric acid was 99.0% and the yield of tris(2-hydroxyethyl) isocyanurate was 82.5 mol %. From this solution, the methyl-cellosolve was removed, and 26.0 g of the crude product thus obtained was recrystallized by adding 24.0 g of ethanol. Upon cooling of the solution, white solid crystals separated out. The precipitate was recovered by filtration and dried. The resulting white solid crystal was found to comprise tris(2-hydroxyethyl) isocyanurate of 99.8% purity.

### Example 4

Except that a commercial heat-resistant anion exchange resin (Daiaion TSA 1200™, product of Mitsubishi Chemical Co.) was used in lieu of the ion exchanger used in Example 1, the reaction was carried out under otherwise the same conditions as in Example 1. From the colorless clear solution available on completion of the reaction, the heat-resistant anion exchange resin was filtered off and the filtrate was analyzed by high performance liquid chromatography. As so analyzed, the conversion rate of isocyanuric acid was 100 mol % and the yield of tris(2-hydroxyethyl) isocyanurate was 72.3 mol %. From this solution the methyl-cellosolve was recovered, and the solution was cooled, whereupon white solid crystals separated out. These crystals were recovered by filtration and dried. The white solid crystal thus obtained was found to comprise tris(2-hydroxyethyl) isocyanurate of 98.5% purity.

### Example 5

Using the heat-resistant anion exchange resin recovered by filtration in Example 4, a repeat experiment was carried out in the same manner as in Example 4. After completion of the reaction, the heat-resistant anion exchange resin was filtered off and the filtrate was analyzed by high performance liquid chromatography. As so analyzed, the conversion rate of isocyanuric acid was 97.5 mol % and the yield of tris(2-hydroxyethyl) isocyanurate was 78.0 mol %. From this solution the methyl-cellosolve was recovered, and the solution was cooled, whereupon white solid crystals separated out. These crystals were recovered by filtration and dried. The white solid crystal thus obtained was found to comprise tris(2-hydroxyethyl) isocyanurate of 99.0% purity.

The crystals obtained were subjected to thermal analysis under the same conditions as in Example 2. The loss of mass after 10 minutes' incubation at 250°C was 1.0% and the loss of mass after arrival at 300°C was 1.7%.

### Comparative Example 1

The addition reaction of ethylene oxide to isocyanuric acid was carried out without using an ion exchanger.

Specifically, an autoclave equipped with a thermometer, pressure gauge, and stirrer, etc. was charged with 9.7 g of isocyanuric acid and 37.5 g of N,N-dimethylformamide, and 9.9 g of ethylene oxide was continuously introduced in a nitrogen atmosphere at 120°C. Thereafter, the reaction mixture was matured at 120°C for 6 hours to synthesize tris(2-hydroxyethyl) isocyanurate. The light-yellow clear solution available on completion of the reaction was analyzed by high performance liquid chromatography. As so analyzed, the conversion rate of isocyanuric acid was 88.4 mol % and the yield of tris(2-hydroxyethyl) isocyanurate was 72.9 mol %. The N,N-dimethylformamide was recovered from this solution and the solution was cooled, whereupon white solid crystals separated out. However, because the system was a yellow viscous fluid, it could hardly be filtered and, therefore, recrystallization from ethanol was carried out. The resulting crystals were recovered by filtration and dried. The white solid crystal thus obtained was found to comprise tris(2-hydroxyethyl) isocyanurate of 98.0% purity. Thermal analysis under the same conditions as in Example 2 revealed that the loss of mass after 10 minutes' incubation at 250°C was 49.5% and the loss of mass after arrival at 300°C was 67.5%. Thus, the product was inferior to that obtained by the method using the ion exchanger in yield, purity and heat resistance.

### Comparative Example 2

Except that the ion exchanger was not used, the addition reaction of ethylene oxide to isocyanuric acid was carried out at 90°C in otherwise the same manner as in Example 1. After completion of the reaction, the unreacted starting material isocyanuric acid was filtered off and the filtrate was analyzed by high performance liquid chromatography. The yield of tris(2-hydroxyethyl) isocyanurate was found to be 1.6 mol %.

### Comparative Example 3

Except that the reaction temperature was changed to 120°C in order to make up for low activity, the addition reaction of ethylene oxide to isocyanuric acid was carried out under otherwise the same conditions as in Comparative Example 2. After completion of the reaction, the unreacted starting material isocyanuric acid was filtered off and the filtrate was analyzed by high performance liquid chromatography. The yield of tris(2-hydroxyethyl) isocyanurate was found to be 26.9 mol %.

### Catalyst preparation method 2

A 1L separable flask equipped with a stirrer, reflux condenser, water separator, reduced pressure maintaining apparatus, thermometer, nitrogen gas inlet tube, and dropping funnel was charged with 350 mL of toluene and 50 mL (12.5 vol. % in medium) of liquid paraffin, and 0.64 g of sorbitan monopalmitate and 0.21 g of ethylcellulose were added and dissolved as dispersion stabilizers at 40°C. In this procedure, the dissolved oxygen was removed by blowing nitrogen gas into the flask.

Separately, 41.74 g of 65% aqueous solution of diallyldimethylammonium chloride (DADMAC), 8.26 g of N,N,N', N'-tetraallyldipiperidylpropanium dichloride (TADPPC), and 20.78 g of water were mixed and dissolved in a 100 mL beaker, and a mixture of 0.32 g of the polymerization initiator "V-50" and 2.4 g of water was added (monomer concentration 48.2%, DADMAC/TADPPC = 90/10 mol %).

This solution was added dropwise to a reaction vessel over 30 minutes at 30°C and, after completion of dropping, the reaction was conducted at 55°C for 4 hours and then at 75°C for 3 hours. In the course, when 2 hours had elapsed, the stirrer speed was increased from 220 rpm to 250 rpm and 50 mL (22.2 vol. % in the medium) of liquid paraffin was further added dropwise. Furthermore, when the temperature was increased from 55°C to 75°C, 50 mL of toluene was further added (liquid paraffin concentration in medium: 20 vol. %) and the polymerization reaction was carried out with reducing the internal pressure and removing the distilled water.

After a predetermined time of reaction, the reaction system was cooled and the product particles were separated by suction filtration. The particles filtered off were washed with 800 mL of methanol for 30 minutes for a total of 3 times and dried under reduced pressure at 60°C overnight to give 32.9 g of dried particles. The dried particles thus obtained were separated particles with a diameter of about 0.2 mm. The particles constitute an ion exchanger having the structure indicated in the above reaction scheme(1).

### Example 6

Using the ion exchanger obtained under Catalyst preparation method 2, the addition reaction of ethylene oxide to succinimide was carried out.

Specifically, an autoclave equipped with a thermometer, pressure gauge, stirrer, etc. was charged with 1.0 g of the ion exchanger, 7.5 g of succinimide, and 12.5 g of 1,4-dioxane, and in a nitrogen atmosphere, 3.4 g of ethylene oxide was introduced at 80°C. Thereafter, the reaction mixture was matured at 80°C for 3.5 hours to synthesize 2-hydroxyethylsuccinimide. From the colorless clear solution available on completion of the reaction, the ion exchanger was filtered off and the filtrate was analyzed by gas chromatography. As so analyzed, the conversion rate of succinimide was 95.9 mol % and the yield of 2-hydroxyethylsuccinimide was 90.6 mol %.

### Example 7

Using the ion exchanger obtained under Catalyst preparation method 2, the addition reaction of ethylene oxide to 2-pyrrolidone was carried out.

Specifically, an autoclave equipped with a thermometer, pressure gauge, stirrer, etc. was charged with 1.0 g of the ion exchanger and 20.0 g of 2-pyrrolidone, and in a nitrogen atmosphere, 5.2 g of ethylene oxide was introduced at 80°C. Thereafter, the reaction mixture was matured at 80°C for 2.5 hours to synthesize 2-hydroxyethylpyrrolidone. From the colorless clear solution available on completion of the reaction, the ion exchanger was filtered off and the filtrate was analyzed by gas chromatography. As so analyzed, the conversion rate of pyrrolidone was 57.3 mol % and the yield of 2-hydroxyethylpyrrolidone was 46.0 mol %.

The present application claims priority under 35 U.S.C. § 119 to Japanese Patent Application No.2001-399983, filed December 28, 2001, entitled "PROCESS FOR PRODUCING N-HYDROXYALKYL COMPOUND, AND TRIS(2-HYDROXYETHYL) ISOCYANURATE COMPOSITION."
The contents of that application are incorporated herein by reference in their entirety.

## Claims

1. A process for producing an N-hydroxyalkyl compound comprising the reaction of an amido or imido group-containing compound and an epoxy compound in the presence of a catalyst
wherein the catalyst is a solid ion exchanger.

2. The process for producing an N-hydroxyalkyl compound according to Claim 1
wherein the solid ion exchanger comprises an organic macromolecular compound essentially having a hetero atom in a main chain and/or a crosslinking site not having a hydrogen atom attached to any of the hetero atom,
the main chain of the organic macromolecular compound predominantly comprising a repeating unit having a cyclic quaternary ammonium salt structure.

3. A tris(2-hydroxyethyl) isocyanurate composition obtainable by the process for producing an N-hydroxyalkyl compound according to Claim 1 or 2.
